# EUROPEAN PATENT SPECIFICATION

(11) **EP 0 357 786 B1**
(45) Date of publication and mention of the grant of the patent: **15.05.1996**
(21) Application number: 89901756.0
(22) Date of filing: 27.01.1989
(51) Int. Cl.: G01N 33/546, G01N 33/553, G01N 33/551

(54) **METHOD FOR ASSAYING ANTIGEN OR ANTIBODY**
TESTVERFAHREN FÜR ANTIGENE ODER ANTIKÖRPER
PROCEDE D'ANALYSE D'ANTIGENE OU D'ANTICORPS

(30) Priority: 29.01.1988 JP 16848/88
(43) Date of publication of application: 14.03.1990
(73) Proprietor: Mitsubishi Chemical Corporation, Chiyoda-ku Tokyo (JP)
(72) Inventor: OGURA, Minoru, Yokohama-shi Kanagawa 233 (JP); SAWAI, Masanobu, Yamato-shi Kanagawa 242 (JP); ITO, Michio, Yokohama-shi Kanagawa 241 (JP); KOHNO, Hideki, Kawasaki-shi Kanagawa 213 (JP)
(74) Representative: Gutmann, Ernest
(86) International application number: JP8900084
(87) International publication number: WO8907270

(56) References cited:
- JP-A- 6 379 070
- JP-A-60 177 265
- JP-A-61 128 168
- JP-A-62 118 255
- JP-A-62 287 159
- JP-A-63 106 559
- JP-A-63 108 265
- JP-A-63 188 766
- JP-A-63 290 964
- US-A- 4 115 535

## Description

### Method for the determination of an amount of antigen and antibody

### Technical Field:

The present invention relates to a method for the determination of an amount of an antigen and/or antibody based on antigen-antibody reaction. It relates particularly to a method of qualitatively or quantitatively determining, by utilizing antigen-antibody reaction, an amount of proteins and substances related thereto contained in such clinical laboratory specimens as serum, plasma and urine in the medical field.

### Background Art:

Currently in the field of immunological diagnosis, four assay methods are commonly used, i.e. radioimmunoassay (RIA), enzyme immunoassay (EIA), turbidimetric immunoassay (TIA) and latex agglutination reaction assay or latex particle immunoassay (LPIA).

Among them, there are methods wherein an amount of an antigen or antibody in a sample may be determined by allowing an antibody or antigen on insoluble carrier particles such as latex particles to react with the antigen or antibody in the sample, i.e.:
(a) After the aggregates which had generated in the reaction have precipitated (usually in 1 to 2 days), the turbidity of the supernatant is measured to detect an amount of unreacted latex particles, so that the amount of the antigen or antibody in the sample may be determined (for example, N. Dezelic et al., Croatica Chemica Acta, 42, 457 (1970));
(b) The reaction mixture, without separation of aggregates therefrom, is subjected to measurement of the intensity of scattered or transmitted light upon exposure to light of a specific wavelength, so that the amount of the antigen or antibody in the sample may be determined from changes of the measured intensity of scattered or transmitted light (for example, Japanese Patent Publication No. 58-11575).

However, a long period of time is required between initiation of the reaction and the measurement in the method (a), while the method (b) entails a problem that it is not necessarily adequate for the measurement of substance requiring a highly sensitive assay.

Besides, there has been proposed an assay method for an antibody or antigen by utilizing antigen-antibody reaction with an antigen or antibody on insoluble carriers, in which a part of the insoluble carriers are magnetic substance (for example, Japanese Patent Application Laying Open (KOKAI) No. 61-128168). In this conventional method, nevertheless, a reaction product has to be separated by taking advantage of magnetism of the carriers and an amount of a preconjugated fluorescent dye or enzyme in the reaction product is measured, thus requiring cumbersome procedure.

Meanwhile, an assay method designed to analyze a component in a multiplicity of samples has recently been proposed and is now in widespread use. Such assay method is characterized by allowing, for example, coloring reactions in a number of wells of a plate and by directly determining an optical density of the reaction mixture in each well.

For example, a so-called "microtiter plate" of about 8 cm × 12 cm possesses 96 wells in which 96 samples can be assayed at a time, and their optical density may be measured within a matter of 30 to 40 seconds by means of an electronic reader for exclusive use. If antigen-antibody reaction by latex aggregation is carried out according to the above method (a) by using such apparatus, the aggregates having precipitated on the bottom of the well will intercept a light path, making an optical measurement impossible. In such case, therefore, the optical density is to be measured sideways (which is, however, nearly impracticable) or to be measured only after transferring each reaction mixture to a suitable vessel.

Lastly, Giaever et al, in US patent 4,115,535 used a mixture of different kinds of particles having different properties: the first one provides a property facilitating separation from the solution while the second one provides a property facilitating detection. Both kinds of these particles agglutinate in the presence of the protein to be detected, and the determination of the amount of the second kind of particles in the agglutinates reflects the amount of the protein to be directed in the sample. The method of Giaever et al needs a separation step of the first kind of particles from the liquid sample and the use of an apparatus to detect the detectable property of the second kind of particle in the agglutinate.

### Disclosure of the Invention:

The Present invention relates to :
1. A method for the determination of an amount of an antigen or an antibody or a mixture thereof contained in a liquid medium, comprising the reaction to equilibrium of this antigen, antibody or mixture thereof with insoluble carrier particles coated with antibody or antigen specific for the antigen or antibody to be determined, characterized :
   - in that said insoluble carrier particles are constituted of a mixture of magnetic substance-containing particles and non-magnetic particles containing possibly a colored dye,
   - in that one applies a magnetic field to separate reacted and unreacted magnetic substance-containing particles,
   - and in that one detects the remaining insoluble carrier particles not containing the magnetic substance, by a turbidimetric method, and compares the result obtained with a standard calibration curve.

The method for the determination of an amount of an antigen or antibody provided by the present invention will now be described in detail below.

As the insoluble carrier containing the magnetic substance, organic polymer material and inorganic fine powder may be used.

Organic polymer latex obtained by emulsion polymerization such as polystyrene and styrene-butadiene copolymer particles may be applicable as the organic polymer material, among which polystyrene latex is particularly useful. As the inorganic fine powder, ceramic fine particles, silica, alumina silica-alumina, metal fine powder and charcoal powder may be used.

For use as the magnetic substance to be contained in the insoluble carrier particles, iron and magnetic iron oxide are preferable and carrier particles to be used are prepared so as to contain 5 to 100% of such magnetic substance.

The insoluble carrier particles of 0.1 to 5 µm in diameter are used, and those of between 0.2 and 2 µm in diameter are preferable.

The above-described organic polymer material and inorganic fine powder may be used as the magnetic substance free insoluble carrier as well, and those which, when dispersed in a liquid medium, do not spontaneously precipitate but remain suspended in the liquid medium until being subjected to optical measurement, are preferable.

Furthermore, as the insoluble carrier not containing the magnetic substance, those red, blue, green, yellow or other color may also be used.

The insoluble carrier particles in any of these colors are used in the measurement of two or more kinds of antibodies or antigens. In case where antigen A and antigen B, for example, are to be measured simultaneously, a mixture of insoluble carrier particles sensitized with an antibody for antigen A (antibody A) and those sensitized with an antibody for antigen B (antibody B) is used as the magnetic substance-containing insoluble carrier particles. As the magnetic substance-free insoluble carrier particles, i.e., the insoluble carrier particles not containing the magnetic substance, on the other hand, a mixture of antibody A-sensitized yellow insoluble carrier particles and antibody B-sensitized blue insoluble carrier particles is used.

In case where neither antigen A nor antigen B is present in an aqueous medium to be measured, therefore, both the yellow and blue insoluble carrier particles will remain in the medium, making the reaction mixture green in appearance. Where antigen A is present in abundance with the absence of antigen B, the reaction mixture will appear blue; and the reaction mixture will appears yellow in a reverse situation. Where both antigens A and B are present in a certain ratio, the reaction mixture will show a corresponding combined color of yellow and blue.

The colored insoluble carrier particles may be prepared by incorporating any of dyes with various colors into organic polymer particles or inorganic fine powder particles, by coating the surface of the particles with the above dye by means of physical adsorption or chemical coupling, or by holding a protein such as albumin by which the dye has been carried by physical adsorption or chemical coupling onto the insoluble particle surface.

The method by which antibody or antigen is carried on the insoluble carrier particles is commonly applied to the magnetic substance-containing insoluble carrier particles and to the magnetic substance-free insoluble carrier particles; it is effected by physical adsorption or chemical coupling to the insoluble carrier particles of an antibody or antigen against the antigen or antibody in a test sample to be determined.

The antibody to be carried on the particles may be γ-globulin, IgG, IgM, F(ab')₂, F(ab') and the like. In case that antigen is carried on the particles, cell fragments, haptens, antigenic proteins, immunocomplexes and the like may be used as the antigen.

The amount of the antigen or antibody to be carried on the particles is usually from 0.01 to 10 mg/mℓ, preferably 0.05 to 1 mg/mℓ.

The insoluble carrier particles carrying antigen or antibody are dispersed and suspended at a concentration between 0.01 and 10% by weight in an aqueous solvent such as 0.1M phosphate buffer (pH 7) or 0.1M Tris-HCℓ buffer (pH 8.0).

The insoluble carrier particles containing the magnetic substance may be blended with the magnetic substance-free insoluble carrier particles at a ratio of between 1:20 and 20:1, preferably between 1:4 and 4:1. The present method is further described in detail below:
(1) The method in which the sensitized insoluble carrier particles containing the magnetic substance and those not containing the magnetic substance are preliminary mixed:
   At first, the sensitized insoluble carrier particles containing the magnetic substance are mixed with those not containing the magnetic substance in the above combining ratio.
   The resulting suspension mixture is then incubated for reaction with antigen or antibody contained in a sample such as plasma, serum or urine (hereinafter referred to as a specimen). The specimen may be diluted with a buffer solution such as 0.1M Tris-HCℓ buffer (pH 8.0) or 0.1M phosphate buffer (pH 7). The incubation is carried out at room temperature until a state of equilibrium is reached. The incubation may be performed in a thermostat at 25-50°C, instead, in order to increase the rate of the above reaction.
   It usually takes about 30 minutes to attain equilibrium of the reaction by incubation at room temperature.
(2) The method in which the magnetic substance containing, sensitized insoluble carrier particles or the magnetic substance-free, sensitized insoluble carrier particles is reacted with a specimen, followed by the addition of the magnetic substance-free, sensitized insoluble carrier particles or the magnetic substance-containing, sensitized insoluble carrier particles to the reaction mixture:
   Firstly, the sensitized insoluble carriers containing the magnetic substance (or those not containing the magnetic substance) are allowed to react with a specimen undiluted or diluted with a buffer solution. This incubation is carried out usually at room temperature for about 10 minutes. These conditions may vary depending on the antigen or antibody to be detected. A period of the incubation is selected such that a state of the equilibrium of the antigen-antibody reaction has not been attained yet between the antibody or antigen loaded on the magnetic substance-containing insoluble carrier particles (or magnetic substance-free insoluble carrier particles) and the antigen or antibody present in the specimen. Subsequently, the magnetic substance-free insoluble carriers (or the magnetic substance-containing sensitized insoluble carriers) are added at the above-mentioned combining ratio to the reaction mixture, which is then further incubated for antigen-antibody reaction at room temperature until a state of equilibrium is reached. The incubation may be performed in a thermostat at 25-50°C, instead, in order to hasten the reaction.

The reaction, whether according to the method (1) or to the method (2) above, is carried out usually in a spectrophotometric cuvette or in a well of microtiter plate. When the reaction has reached a state of equilibrium, aggregates of the magnetic substance-containing sensitized insoluble carrier particles and the magnetic substance-free sensitized insoluble carrier particles as well as unreacted magnetic substance-containing sensitized insoluble carrier particles are collected at such a site as not intercepting a measuring light path through a reaction vessel either by applying a magnetic field from outside of the vessel, or by placing or inserting tiny magnet balls or rods into the reaction mixture. A magnet to be used for this purpose may be a permanent magnet, electromagnet and the like. Eventually, the magnetic substance-free sensitized insoluble carriers remains suspended in the reaction medium. The amount of the carriers remaining in the reaction medium is dependent on the amount of antigen present in the specimen assayed for antigen, or on that of antibody present in the specimen assayed for antibody. This amount of carriers remaining in the reaction medium may be determined by measurement of scattered light intensity or the absorbance of light at a wavelength of between 0.3 and 1.0 µm from the outside of the vessel. In the present invention, the concentration of antigen or antibody in a given specimen can be quantitatively determined from the absorbance or the scattered light intensity with reference to a calibration curve which has been previously constructed on the basis of values determined for the absorbance or scattered light intensity of specimens containing known and different concentration levels of the antigen or antibody to be measured.

Alternatively, it is also practicable with the method of the present invention to qualitatively decide the amount of antigen or antibody by visual detection of the amount of the magnetic substance-free insoluble carrier particles remaining in the reaction medium.

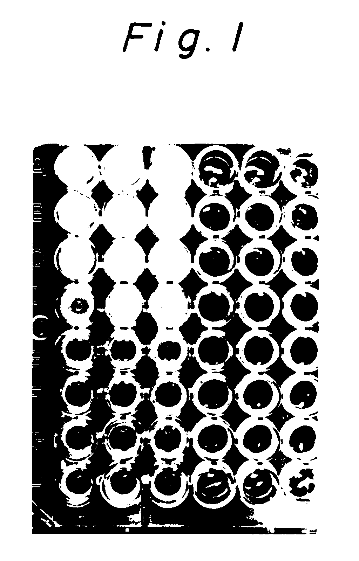

Shown in Fig. 1 are wells with a V-shaped bottom of a microtiter plate, where reaction mixtures with serial two-fold dilutions of an antigen specimen in each row of the plate were subjected to a magnetic field from the bottom of the plate after incubation. The magnetic substance-containing carriers and aggregated carriers have rapidly precipitated on the V-shaped bottom of the wells. Consequently, the resulting supernatant is completely clear in wells of the specimens with high concentrations of the antigen and, with decreasing concentration of the antigen, the supernatant progressively increases in opacity so that the precipitates on the V-shaped bottom of the well (the precipitates actually appear brownish to yellowish) become increasingly obscured. It is then without difficulty to visually discern between plus and minus as to the amount of the antigen according to the degree of visibility of the precipitates in the well. Depending on circumstances, it may be also possible to quantitate the antigen or antibody up to a certain level with the use of controls prepared by a standard.

Meanwhile, it has been generally thought that it is difficult to apply monoclonal antibodies to the latex agglutination assay. In the method of the present invention, however, antigen which may react with a monoclonal antibody as well as with a polyclonal antibody can be qualitatively or quantitatively measured in the same manner as that described above, by using magnetic substance-free insoluble carrier particles sensitized with the monoclonal antibody and magnetic substance-containing insoluble carrier particles sensitized with the polyclonal antibody.

Thus, the present method is distinct from the prior art using magnetic substance-containing particles in the following respects:
1. Since the present method does not involve the use of any labeled insoluble carrier particles, such procedures are unnecessary as of removing the supernatant after magnetically collecting within the reaction vessel the magnetic substance particles and aggregates which contain magnetized particles and of washing. That is, the procedures are simple, and the entire process from reaction to measurement can be accomplished within the same reaction vessel. This constitutes a great advantage of the present method for automation of the assay procedure.
2. The method of the present invention does not use any fluorescent dyes or radioisotopes, and hence does not need any specialized apparatus or installation. Measurement can be accomplished with an ordinary spectrophotometer or microplate reader.
3. According to the present invention, the magnetic substance-free insoluble carrier particles remaining in the reaction medium are detected, but the aggregated particles are not detected. That is, the absorbance or scattered light intensity to be measured is maximal in the absence of the antigen or antibody in the specimen, and the absorbance or scattered light intensity of the magnetic substance-free insoluble carrier particles diminishes progressively with the increasing amount of the antigen or antibody in the specimen. Accordingly, the concentration of the magnetic substance-free insoluble carrier particles may be adjusted beforehand in order to carry out the measurement of the absorbance or scattered light intensity at the maximal sensitivity. This makes it possible to detect the antigen or antibody with a high sensitivity, particularly at a low concentration in the vicinity of a blank value.
   Furthermore, the present method is non-time-consuming since the aggregated particles can be promptly removed, thereby providing a substantial reduction of time required for the measurement.

### Brief Description of Drawing:

Fig. 1 shows a microtiter plate with V-shaped bottomed wells, each row of which contains the magnetic substance-containing insoluble carrier particles and aggregates comprising thereof, which have been magnetically precipitated on the bottom of the wells after incubation with serial two-fold dilutions of human chorionic gonadotropin (HCG) as a sample. In Fig. 1, the wells of row H hold reaction mixtures containing 9000 mIU/mℓ of HCG and those of successive rows hold reaction mixtures containing serial two-fold dilutions of HCG ranging down to nil in row A.

### Best Mode for Carrying Out the Invention:

The present invention will hereinafter be described more particularly with reference to the Examples. However, the Examples are merely intended to be illustrative and should not be construed as limiting the scope of the invention.

### Example 1: Detection of AFP

### (Preparation of reagents)

### ·Preparation of anti-AFP-sensitized latex containing the magnetic substance

To 1 mℓ of a 1% suspension in 0.1M Tris-HCℓ buffer (pH 8.0) of latex particles which had been separated from 10% (w/v) of 0.7-µm magnetic latex (Estapor SML266; Rhône-Poulenc), 1 mg of an anti-AFP specific antibody obtained by immunization of animals with human α-fetoprotein (AFP) was added, and the resulting mixture was stirred for about 1 hour to sensitize the latex with the antibody. The mixture is then centrifuged at 16,000 rpm for 10 minutes, and the supernatant was discarded. The latex thus separated was redispersed in 0.1M Tris-HCℓ buffer (pH 8.0) supplemented with 0.3% bovine serum albumin. This latex suspension was stirred for 1 hour, then recentrifuged at 16,000 rpm for 10 minutes, and the resulting supernatent was removed. The latex precipitate was redispersed in 0.1M phosphate buffer (pH 7).

### ·Preparation of anti-AFP-sensitized latex not containing the magnetic substance

To 1 ml of a 1% suspension in 0.1M Tris-HCℓ buffer (pH 8.0) of latex which had been separated from 10% (w/v) of 0.497-µm polystyrene latex (Dow Chemical), 1 mg of the anti-AFP specific antibody was added and the resulting mixture was stirred for about 1 hour to sensitize the latex with the antibody. The mixture was then centrifuged at 16,000 rpm for 10 minutes, the resulting supernatant was discarded. The latex thus separated was redispersed in 0.1M Tris-HC buffer (pH 8.0) containing 0.3% bovine serum albumin. This latex suspension was stirred for 1 hour, then recentrifuged at 16,000 rpm for 10 minutes. The resulting supernatant was removed and the latex precipitate was redispersed in 0.1M phosphate buffer (pH 7).

### (Procedure)

One percent suspension of the magnetic substance-containing, anti-AFP-sensitized latex was blended with 1% suspension of the magnetic substance-free, anti-AFP-sensitized latex at a ratio of 1:1, and the mixture was diluted by eight times in 0.1M phosphate buffer. This diluted mixture was designated as a latex preparation reagent.

Serial two-fold dilutions of AFP standard (250 ng/mℓ; DIA IATRON) were prepared by using AFP-free human serum to serve as a specimen.

Fifty µℓ of the specimen, 50 µℓ of buffer solution (0.1M Tris-HCℓ, pH 8.0) and 50 µℓ of the latex preparation reagent were added to each well of a 96-well, flat-bottomed microtiter plate (Flexible Assay Plate; Falcon), thoroughly mixed, and incubated for 30 minutes at room temperature. Subsequently, a permanent magnet was applied onto the side of the wells of the microtiter plate from the outside thereof to collect the aggregates of the magnetic substance-containing latex and unreacted magnetic substance-containing latex at the corresponding inner side of the wells. This collecting process usually took about 5 minutes. Then, the concentration of the magnetic substance-free insoluble carrier particles remaining in the liquid medium in the well of the microtiter plate was spectrophotometrically determined by measuring the optical density of the liquid in the wells at a wavelength of 620 nm with a Microplate Reader (NJ2000; Intermed Japan Inc.).

Calibration data on optical density and AFP concentration are presented in Table 1.

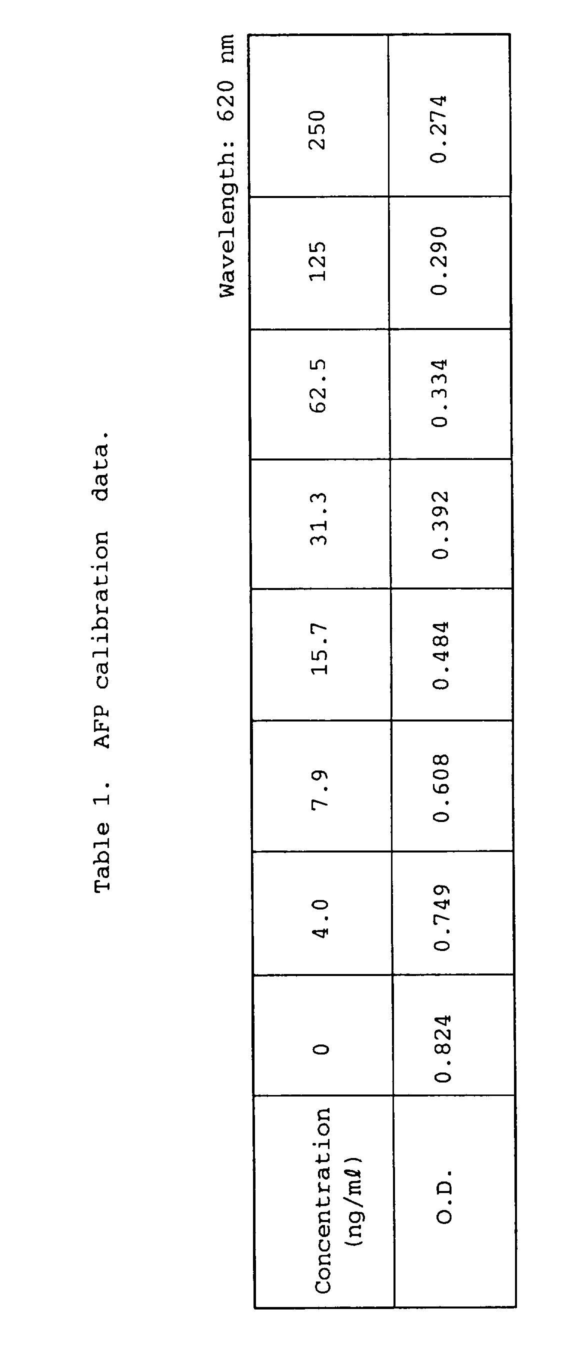

### Example 2: Detection of E. coli thermolabile enterotoxin

### (Preparation of reagents)

### ·Preparation of anti-enterotoxin-sensitized latex containing the magnetic substance

As Vibrio cholerae enterotoxin is immunologically common in antigenecity with Escherichia coli thermolabile enterotoxin, 0.1 mg/ml of an anti-E. coli thermolabile enterotoxin antibody obtained by immunizing animals with V. cholerae enterotoxin was added to 1 mℓ of a 1% suspension in 0.1M Tris-HCℓ buffer (pH 8.0) of latex which had been separated from 10% magnetic substance-containing latex (Estapor SML266; Rhône-Poulenc). The resulting mixture was stirred for about 1 hour to sensitize the latex with the antibody, then centrifuged at 16,000 rpm for 10 minutes. The resulting supernatant was discarded. The latex precipitate was redispersed in 0.1M Tris-HCℓ buffer (pH 8.0) containing 0.1% bovine serum albumin, stirred for about 1 hour, and centrifuged at 16,000 rpm for 10 minutes. The resulting supernatant was removed and the precipitate was again redispersed in 0.1M phosphate buffer. ·Anti-enterotoxin-sensitized latex not containing the magnetic substance

A sensitized latex in a commercially available assay kit (Kit for Detection of V. cholerae Enterotoxin and E. coli Thermolabile Enterotoxin; Denka Seiken Co., Ltd.) was used as a magnetic substance-free sensitized latex.

### (Procedure)

One percent suspension of the magnetic substance-containing sensitized latex was diluted to 1:8 in 0.1M phosphate buffer (pH 7.0), and blended at a ratio of 1:2 with the magnetic substance-free sensitized latex. This mixture was designated as a latex preparation suspension.

Serial two-fold dilutions of a 40-ng/mℓ solution of enterotoxin in 0.1M Tris-HCℓ buffer (pH 8.0) were prepared by using 0.1M Tris-HCℓ buffer (pH 8.0) as a diluent, serving as an assay specimen.

Fifty µℓ of the specimen and 100 µℓ of the latex preparation suspension were added to each well of a 96-well, flat-bottomed microtiter plate (Flexible Assay Plate; Falcon), thoroughly mixed, and incubated at room temperature for 30 minutes.

Subsequently, a permanent magnet was applied onto the side of the wells of the microtiter plate from the outside thereof to collect the aggregates of the magnetic substance-containing latex and unreacted magnetic substance-containing latex at the corresponding inner side of the wells. This collecting process usually took about 5 minutes. The optical density of the liquid in the wells of the microtiter plate was then determined at a wavelength of 620 nm with a Microplate Reader (NJ2000; Intermed Japan Inc.). Calibration data on optical density and the concentration of enterotoxin are presented in Table 2.

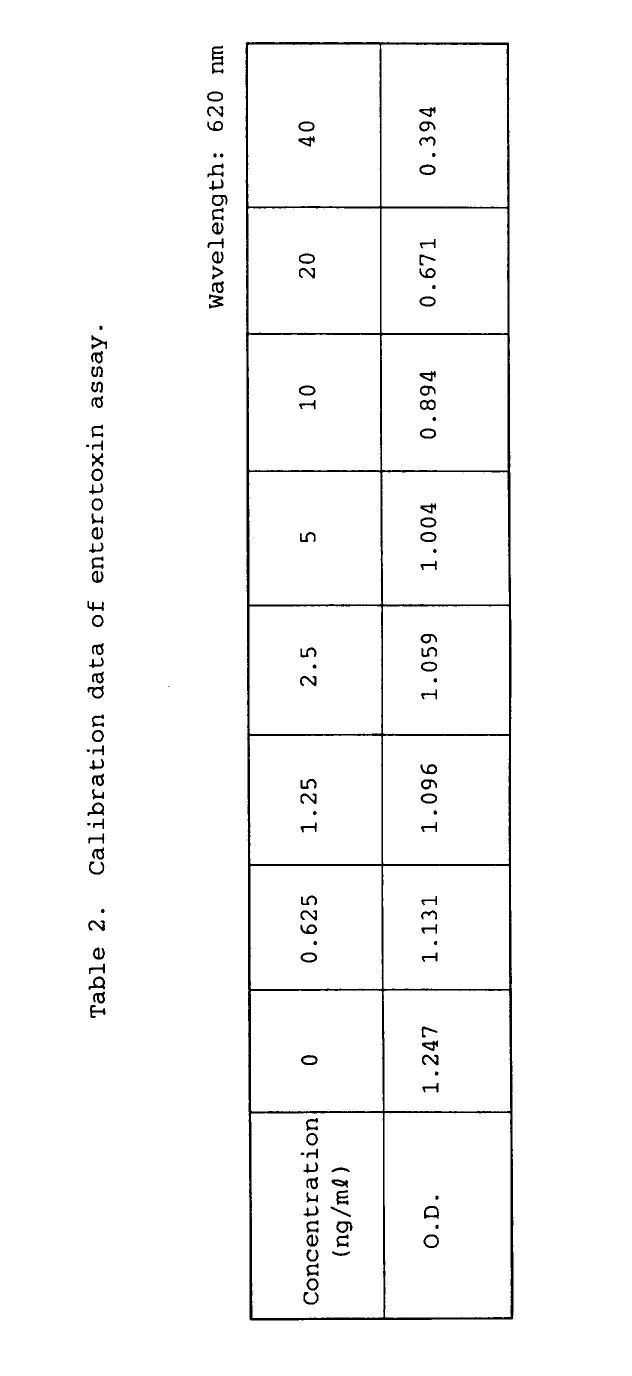

### Example 3: Simultaneous detection of AFP and CEA

### (Preparation of reagents)

### 3-1 Preparation of anti-AFP-sensitized latex containing the magnetic substance

To 1 mℓ of a 1% suspension in 0.1M Tris-HCℓ buffer (pH 8.0) of latex which had been separated from 10% (w/v) magnetic latex of 0.7 µm mean particle diameter (Estapor SML266; Rhone-Poulenc), 1 mg of an anti-AFP specific antibody obtained by immunizing animals with human α-fetoprotein (AFP) was added; and the resulting mixture was stirred for about 1 hour to sensitize the latex with the antibody. It was then centrifuged at 16,000 rpm for 10 minutes, the supernatant was removed, and the latex precipitate was redispersed in 0.1M Tris-HCℓ buffer (pH 8.0) containing 0.3% bovine serum albumin and stirred for 1 hour. After recentrifugation at 16,000 rpm for 10 minutes, the supernatant was discarded and the precipitate was again redispersed in 0.1M phosphate buffer (pH 7).

### 3-2 Preparation of anti-CEA-sensitized latex containing the magnetic substance

To 1 mℓ of a 1% suspension in 0.1M Tris-HCℓ buffer (pH 8.0) of latex which had been separated from 10% (w/v) magnetic latex of 0.7 µm mean particle diameter (Estapor SML266; Rhône-Poulenc), 0.5 mg of an anti-CEA specific antibody obtained by immunizing animals with human carcinoembryonic antigen (CEA) was added, and the resulting mixture was stirred for about 1 hour to sensitize the latex with the antibody. It was then centrifuged at 16,000 rpm for 10 minutes, the supernatant was removed, and the latex precipitate was redispersed in 0.1M Tris-HCℓ buffer (pH 8.0) containing 0.1% bovine serum albumin and stirred for 1 hour. After recentrifugation at 16,000 rpm for 10 minutes, the supernatent was discarded and the latex precipitate was again redispersed in 0.1M phosphate buffer (pH 7).

### 3-3 Preparation of anti-AFP-sensitized latex not containing the magnetic substance

To 1 mℓ of a 1% suspension in 0.1M Tris-HCℓ buffer (pH 8.0) of latex which had been separated from 10% (w/v) blue polystyrene latex of 0.45-0.55 µm particle diameter (Estapor K050 Blue; Rhône-Poulenc), 1 mg of an anti-AFP specific antibody was added, and the resulting mixture was stirred for about 1 hour to sensitize the latex with the antibody. It was then centrifuged at 16,000 for 10 minutes, the supernatant was removed, and the latex precipitate was redispersed in 0.1M Tris-HCℓ buffer (pH 8.0) containing 0.3% bovine serum albumin and stirred for 1 hour. After recentrifugation at 16,000 rpm for 10 minutes, the supernatent was discarded and the latex precipitate was again redispersed in 0.1M phosphate buffer (pH 7).

### 3-4 Preparation of anti-CEA-sensitized latex not containing magnetic substance

To 1 mℓ of a 1% suspension in 0.1M Tris-HCℓ buffer of latex which had been separated from 10% (w/v) yellow polystyrene latex of 0.45-0.55 µm particle diameter (Estapor K050 yellow; Rhône-Poulenc), 0.5 mg of an anti-CEA specific antibody was added, and the resulting mixture was stirred for about 1 hour to sensitize the latex with the antibody. It was then centrifuged at 16,000 rpm for 10 minutes, the supernatant was removed, and the latex precipitate was redispersed in 0.1M Tris-HCℓ buffer (pH 8.0) containing 0.1%/bovine serum albumin and stirred for 1 hour. After recentrifugation at 16,000 rpm for 10 minutes, the supernatant was discarded and the latex precipitate was again redispersed in 0.1M phosphate buffer (pH 7).

Each of the four latex suspensions prepared as described in 3-1, 3-2, 3-3 and 3-4 above was diluted two-fold with 0.1M phosphate buffer (pH 7.0), and equal volumes of these four dilutions were combined and thoroughly mixed to obtain a prepared suspension for simultaneous detection of AFP and CEA.

### (Procedure)

Serial two-fold dilutions of AFP standard (250 ng/mℓ; DIA IATRON) were prepared by using AFP- and CEA-free human serum. Serial two-fold dilutions of CEA standard (160 ng/mℓ; MITSUBISHI KASEI Corp.) were also prepared by using AFP- and CEA-free human serum. A solution containing 125 ng/mℓ of AFP and 80 ng/mℓ of CEA was prepared by combining the AFP standard (250 ng/mℓ) and the CEA standard (160 ng/mℓ) at a ratio of 1:1. This solution was diluted two-fold serially in 0.1M Tris-HCℓ buffer to obtain specimen models containing known concentrations of both AFP and CEA. These three series of two-fold dilutions were subjected as specimens to the assay.

To wells of a 96-well, flat-bottomed microtiter plate (Flexible Assay Plate; Falcon), 50 µℓ of the specimen, 50 µℓ of 0.2M Tris-HCℓ buffer (pH 8.0) and 50 µℓ of the prepared suspension for simultaneous detection of AFP and CEA were added, mixed thoroughly, and incubated for 30 minutes at room temperature. Then, a permanent magnet was applied onto the side of the wells of the microtiter plate from the outside thereof to collect the aggregates of the magnetic substance-containing latex and unreacted magnetic substance-containing latex at the corresponding inner side of the wells. This collecting process took usually about 10 minutes. The microtiter plate was read for detection of AFP and CEA by a visual assessment of the color of the liquid in the wells. Results are shown in Table 3.

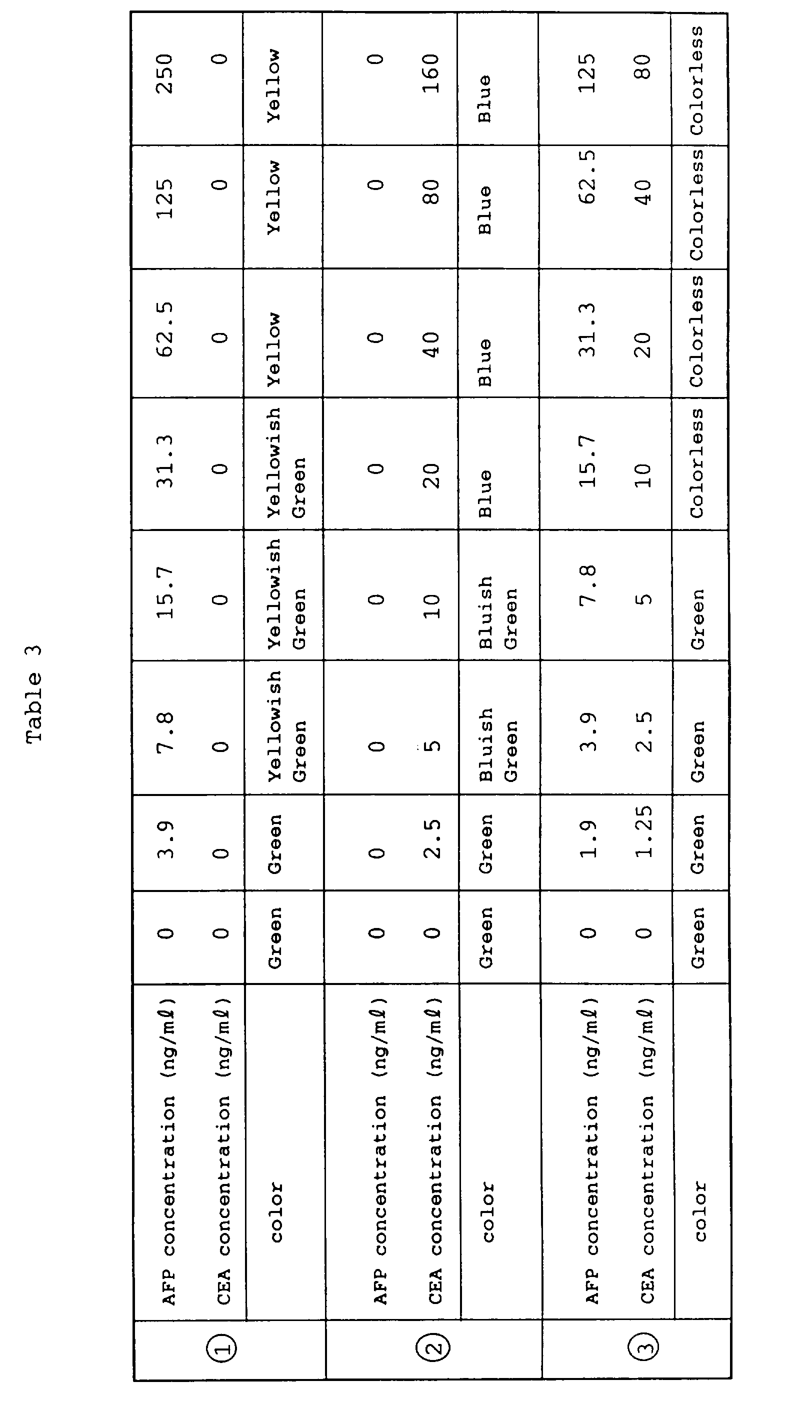

The specimen is positive for AFP if color of the reaction mixture in the well of the microtiter plate is yellow, is positive for CEA if blue, is negative for both AFP and CEA if green, or is positive for both AFP and CEA if colorless.

### Industrial Applicability:

The method for the determination of an antigen and/or antibody provided by the present invention is characterized by that extremely minute quantities of the antibody or antigen can be rapidly measured qualitatively or quantitatively since the aggregates of reacted carriers may be not only drawn in any desired direction but also remain scarcely redispersed on the vessel wall surface and that the procedures of the present method are simple and noncumbersome. It may therefore be used for the measurement of a trace amount of substance in various fields of science such as medicine, biochemistry, hygienics and epidemiology.

## Claims

1. A method for the determination of an amount of an antigen or an antibody or a mixture thereof contained in a liquid medium, comprising the reaction to equilibrium of this antigen, antibody or mixture thereof with insoluble carrier particles coated with antibody or antigen specific for the antigen or antibody to be determined, characterized :
- in that said insoluble carrier particles are constituted of a mixture of magnetic substance-containing particles and non-magnetic particles containing possibly a colored dye,
- in that one applies a magnetic field to separate reacted and unreacted magnetic substance-containing particles,
- and in that one detects the remaining insoluble carrier particles not containing the magnetic substance, by a turbidimetric method, and compares the result obtained with a standard calibration curve.

2. A method according to claim 1, wherein the insoluble carrier particles containing iron and/or magnetic iron oxide as the magnetic substance are used.

3. A method according to claim 1 or 2, wherein material used for the insoluble carrier particles can be selected from organic polymers or inorganic fine powders.

4. A method according to claim 3, wherein polystyrene latex or styrene-butadiene copolymer is used for the insoluble carrier particles.

5. A method according to claim 3, wherein ceramic fine particles, silica, alumina, silica-alumina charcoal powder or metal fine powder are used for the insoluble carrier particles.

6. A method according to claim 1, wherein the insoluble carrier particles containing the magnetic substance and those not containing the magnetic substance are combined at a ratio of between 1:20 and 20:1.

7. A method according to claim 1, wherein the amount of the antigen or antibody in a specimen is qualitatively determined by visual detection of the amount of the remaining insoluble carrier particles not containing the magnetic substance.

## Patentansprüche

1. Verfahren zur Mengenbestimmung eines in einem flüssigen Medium enthaltenen Antigens oder Antikörpers oder eines Gemisches daraus, umfassend die Umsetzung des Antigens, des Antikörpers oder des Gemisches daraus mit unlöslichen Trägerpartikeln, die beschichtet sind mit Antikörpern oder Antigenen, die für das zu bestimmende Antigen oder den zu bestimmenden Antikörper spezifisch sind, bis zur Einstellung des Gleichgewichts, dadurch gekennzeichnet,
daß die unlöslichen Trägerpartikel aus einem Gemisch aus magnetische Substanzen enthaltenden Partikeln und nichtmagnetischen Partikeln, die einen Farbstoff enthalten können, bestehen,
daß ein magnetisches Feld angelegt wird, um die die magnetische Substanz enthaltenden umgesetzten und nicht umgesetzten Partikel abzutrennen,
und daß man die verbleibenden unlöslichen Trägerpartikel, die die magnetische Substanz nicht enthalten, durch eine turbidimetrische Methode nachweist und das Ergebnis mit einer Standardeichkurve vergleicht.

2. Verfahren nach Anspruch 1, wobei die verwendeten unlöslichen Trägerpartikel Eisen und/oder magnetisches Eisenoxid als magnetische Substanz enthalten.

3. Verfahren nach Anspruch 1 oder 2, wobei das für die unlöslichen Trägerpartikel verwendete Material unter organischen Polymeren oder anorganischen feinen Pulvern ausgewählt werden kann.

4. Verfahren nach Anspruch 3, wobei Polystyrollatex oder ein Styrol-Butadien-Copolymer für die unlöslichen Trägerpartikel verwendet wird.

5. Verfahren nach Anspruch 3, wobei keramische Feinpartikel, Siliciumdioxid, Aluminiumoxid, Siliciumdioxid-Aluminiumoxid, Aktivkohle oder feine Metallpulver für die unlöslichen Trägerpartikel verwendet werden.

6. Verfahren nach Anspruch 1, wobei die unlöslichen Trägerpartikel, die die magnetische Substanz enthalten, und diejenigen, die die magnetische Substanz nicht enthalten, in einem Verhältnis von 1:20 bis 20:1 kombiniert werden.

7. Verfahren nach Anspruch 1, wobei die Menge an Antigen oder Antikörper in einer Probe durch visuellen Nachweis der Menge an verbleibenden unlöslichen Trägerpartikeln, die die magnetische Substanz nicht enthalten, bestimmt wird.

## Revendications

1. Procédé de détermination d'une quantité d'un antigène ou d'un anticorps ou d'un mélange de ceux-ci contenu dans un milieu liquide, comprenant la réaction jusqu'à l'équilibre de cet antigène, anticorps ou mélange de ceux-ci avec des particules de support insolubles revêtues d'un anticorps ou antigène spécifique de l'antigène ou de l'anticorps à déterminer, caractérisé :
- en ce que lesdites particules de support insolubles sont constituées d'un mélange de particules contenant une substance magnétique et de particules non magnétiques contenant éventuellement un colorant coloré,
- en ce qu'on applique un champ magnétique pour séparer les particules contenant une substance magnétique ayant réagi et n'ayant pas réagi,
- et ce qu'on détecte les particules de support insolubles restantes ne contenant pas la substance magnétique, par un procédé turbidimétrique, et qu'on compare le résultat obtenu à une courbe d'étalonnage standard.

2. Procédé selon la revendication 1, caractérisé en ce qu'on utilise les particules de support insolubles contenant du fer et/ou de l'oxyde de fer magnétique comme substance magnétique.

3. Procédé selon la revendication 1 ou 2, caractérisé en ce que la matière utilisée pour les particules de support insolubles peut être choisie parmi les polymères organiques ou les poudres minérales fines.

4. Procédé selon la revendication 3, caractérisé en ce qu'on utilise du latex de polystyrène ou du copolymère de styrène-butadiène comme particules de support insolubles.

5. Procédé selon la revendication 3, caractérisé en ce qu'on utilise des particules céramiques fines, de la silice, de l'alumine, de la silice-alumine, de la poudre de charbon ou de la poudre métallique fine comme particules de support insolubles.

6. Procédé selon la revendication 1, caractérisé en ce que les particules de support insolubles contenant la substance magnétique et celles ne contenant pas la substance magnétique sont combinées dans un rapport compris entre 1:20 et 20:1.

7. Procédé selon la revendication 1, caractérisé en ce que la quantité d'antigène ou d'anticorps dans un prélèvement est déterminée qualitativement par détection visuelle de la quantité de particules de support insolubles restantes ne contenant pas la substance magnétique.
